# EUROPEAN PATENT SPECIFICATION

(11) **EP 0 812 182 B1**
(45) Date of publication and mention of the grant of the patent: **28.02.2001**
(21) Application number: 95943164.4
(22) Date of filing: 13.12.1995
(51) Int. Cl.: A61K 7/32, A61K 7/38

(54) **ANTIPERSPIRANT COMPOSITIONS**
SCHWEISSHEMMENDE MITTEL
COMPOSITIONS ANTITRANSPIRATION

(30) Priority: 09.02.1995 GB 9502495
(43) Date of publication of application: 17.12.1997
(73) Proprietor: UNILEVER PLC, London EC4P 4BQ (GB); UNILEVER N.V., 3013 AL Rotterdam (NL)
(72) Inventor: CORREIA, Martinho, Wetherby West Yorkshire LS22 5RB (GB)
(74) Representative: Bryant, Tracey
(86) International application number: EP9504999
(87) International publication number: WO9624326

(56) References cited:
- EP-A- 0 373 424
- EP-A- 0 485 012
- EP-A- 0 499 398
- GB-A- 2 050 162
- US-A- 4 806 338
- HAPPI, vol. 23, no. 2, February 1986, RAMSEY, GB, pages 43-52, XP002004766 A. J. DISAPIO: "new approaches to antiperspirant and deodorant formulation"

## Description

This invention relates to antiperspirant compositions. In particular, it relates to propellant driven aerosol compositions which are capable of being dispensed from a pressurised aerosol container. In particular, it relates to emulsion antiperspirant aerosol compositions, in which the antiperspirant active is dissolved in one phase of an emulsion.

Propellant driven silicone based emulsion products are known for application to the skin, such as in skin care products. For example, in European patent application 93200984.8 (Unilever NV et al), there is described a propellant driven microemulsion product comprising 20-70% by weight of a water in silicone oil microemulsion base, and 30-80% by weight of a volatile diluent/propellant. Such products are applied as a propellant driven aerosol formulation, and form a clear non-running gel on the skin. Such compositions may optionally also comprise active skin care materials, such as deodorant or antiperspirant actives. In practical embodiments of such compositions, the water-in-silicone oil base microemulsion additionally comprises 20-40% by weight of propylene glycol. The main benefit of such compositions is said to be that they form clear gels on the skin, after application.

With regard to a different technical problem, it has long been known that there are problems in incorporating a dissolved antiperspirant active salt, such as an aluminium salt, into a propellant driven aerosol composition containing water. In the presence of water, the aluminium salt generates aluminium ions, which are powerful corrosives. In spite of other measures which may be taken to protect the aerosol container, such as lacquering the inside of the container, a common problem with such compositions is that the aluminium ions attack and corrode the inner surface of the can. In extreme cases this can lead to perforation of the can, or "pinholing". Such products are unsafe for general sale.

It is an object of the invention to provide propellant driven antiperspirant aerosol compositions which contain a dissolved aluminium salt, which have a generally reduced incidence of pinholing of the container, and may therefore be generally safer to use.

It is a further object of the invention to provide antiperspirant aerosol compositions containing dissolved aluminium salts, which provide little or no visible deposits after application to the human skin.

It is yet a further object to the invention to provide propellant driven aerosol antiperspirant compositions which contain a dissolved aluminium antiperspirant active, which have superior sensory properties, and improved feel on the skin after application.

It is yet a further object of the invention to provide propellant driven aerosol emulsion antiperspirant products which have a generally reduced incidence of pinholing of the container, and which may therefore be generally safer to use.

Thus, according to a first aspect of the invention, there is provided a propellant driven emulsion antiperspirant aerosol composition comprising 10-50% of a base and a 50-90% of a propellant, the base being in the form of a water-in-oil emulsion and comprising a dissolved aluminium salt, a volatile silicone, and a silicone surfactant.

Preferably, the base component of the composition comprises 10-40%, more preferably 10-25% by weight of the composition, while the propellant correspondingly preferably comprises 60-90%, more preferably 75-90% by weight of the composition.

By volatile silicone is preferably meant a cyclic or linear polydimethyl siloxane. Preferred cyclic polydimethylsiloxanes have from 3-6 silicon atoms, and a viscosity of less than 10 mm²s⁻¹ (cSt) at 25°C. Preferred linear polydimethylcyloxanes have from 3-9 silicon atoms, and a viscosity of less than 5 mm²s⁻¹ (cSt) at 25°C. Preferred polydimethylsiloxanes are available for example from Dow Corning Corporation under such names as Dow Corning 344, 345 and 200 fluid. Preferably the volatile silicone is present in the formulation at a level of 1-20%, more preferably 1.5-10% by weight total composition (including propellant).

By silicone surfactant is meant an amphiphilic molecule having a lipophilic silicone chain, and a polar head group. Preferred species of silicone surfactants are those comprising dimethicone copolyol silicone surfactants. A convenient way to incorporate the silicone surfactant in the composition is by way of including a silicone based composition comprising a silicone surfactant. Preferred silicone surfactant containing materials include DC3225C fluid, available from Dow Corning, which comprises 90% volatile silicone, and 10% dimethicone copolyol silicone surfactant.

Preferably the silicone surfactant is present in the composition at a level of 0.05-0.5%, more preferably 0.1%-0.4% by weight of the composition.

Water is a further essential component of compositions according to the invention, and may be present at levels of 2-25%, more preferably 2-15% by weight of the composition.

It is highly preferred that the composition additionally comprises 1-10% by weight of a short chain C₁-C₆ monohydric alcohol, for example ethanol.

Propellant driven antiperspirant aerosol compositions according to the invention exhibit remarkably low corrosion problems, including pinholing, in use. In addition, aerosol antiperspirant compositions according to the invention may generate relatively low amounts of visible deposit on application to the skin, and also have remarkably good sensory properties when applied to the skin.

The base component of antiperspirant compositions according to the invention, whilst being in the form of an emulsion, need not be a micro-emulsion; as such, it will not necessarily appear clear, but may be translucent.

Without wishing to be bound by theory, it is suspected that somehow the volatile silicone and silicone surfactant combination conspire to render the aluminium ions in the composition to be less corrosive, thereby causing less pinholing.

In a highly preferred aspect of the invention, the base of the composition additionally comprises 1-10% by weight of an emollient, preferably a C₂-C₄ polyol emollient, such as a glycol emollient. Preferred C₂-C₄ polyol emollients include propylene glycol and glycerol. The presence of such an emollient in the composition has been found to reduce the incidence of white deposits on the skin, when the composition is applied. Other suitable emollients include isopropyl myristate, isomyristyl myristate, and dipropylene glycol. Preferably, the emollient is present in the composition at a level of 0.5-10% by weight, more preferably 1-8% by weight of the composition.

The aluminium salt used in compositions according to the invention may be any antiperspirant active aluminium salt which is suitable for inclusion in a propellant driven aerosol composition. Suitable aluminium salts include aluminium halides, aluminium hydroxyhalides and mixtures thereof. Many such compounds are commercially available, and are frequently used in the art. Preferred aluminium salts include aluminium chlorhydrates and activated aluminium chlorhydrates, such as are described for example in EP6,739 (Unilever NV et al). These may conveniently be obtained commercially as aqueous solutions of salts, for example Reach 301 (aluminium chlorhydrate), or Reach 501 (activated aluminium chlohydrate), ex Reheis, or Aloxicoll L (aluminium chlorhydrate) or Aloxicoll ASL (aluminium sesquichlorohydrate), ex Giulini. Conveniently the aluminium salt is present in the composition at a level of 0.5-25%, more preferably 2-10% by weight of the composition.

Suitable propellants for use in compositions according to the invention are those which enable the composition to be dispensed from an aerosol container. They include liquifiable propellants, such as propane, n-butane, dimethyl ether, and methylene chloride, and can be used individually or in admixture, and non liquifiable propellants, such as carbon dioxide, nitrogen, nitrogen oxides, and air. Particularly suitable are the CAP range of hydrocarbon propellants, especially CAP 30 and CAP 40.

The composition according to the invention may additionally comprise other components commonly found in antiperspirant aerosol compositions. These may include;
- non-volatile silicones, such as polyalkylsiloxanes, polyalkylaryl siloxanes, or polyether siloxane copolymers, for example having a viscosity ranging from 10-100,000mm²s⁻¹ (cSt) at 25°C. Such siloxanes are available for example from Dow Corning as the DC200 series.
- skin feel improvers, such as talc and finely divided polyethylene, for example Accumist B18,
- cosmetically acceptable vehicles, such as anhydrous ethanol and other emollients,
- gelling and thickening agents, such stearyl alcohol or waxes, or silicone gums (for example Q2/1401, available from Dow Corning)
- perfumes and deo perfumes,
- preservatives,
and other cosmetic adjuncts conventionally employed in propellant driven aerosol antiperspirant products.

A further particularly preferred embodiment of the invention is the inclusion in the composition of 0.1-1% by weight of a silicone gum. This minimises billowing of the spray in use, and may act to improve the capture efficiency of the spray on the skin.

A particularly preferred embodiment of the invention involves packaging the composition in an aerosol container made of aluminium. Whilst a benefit can be observed in using compositions according to the invention in other aerosol cans, such as those made from tin plate, superior results can be obtained if the composition is packaged in an aluminium can.

### EXAMPLES

The invention will now be further described by way of example only.

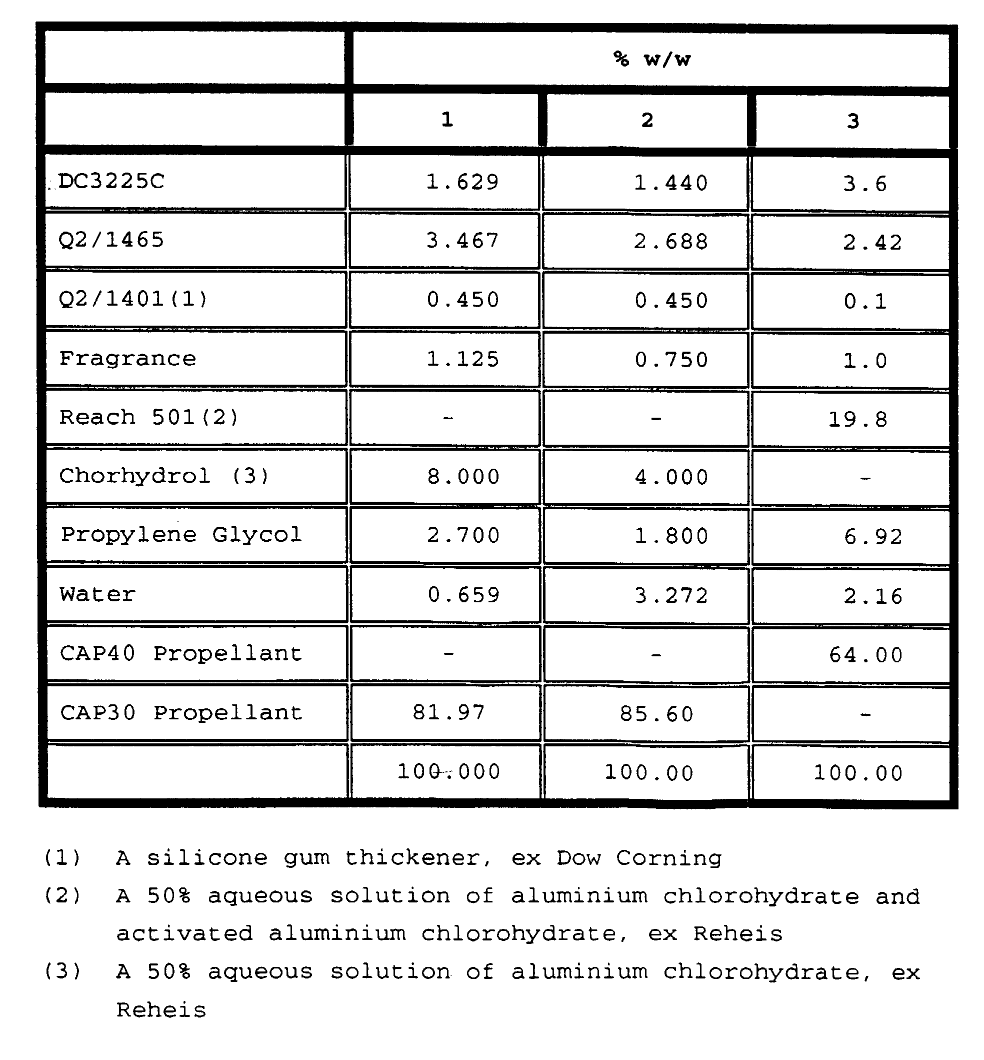

### Preparation Method

Examples 1-3 were prepared according to the same general method.

Thus, the DC3225C fluid, Q2/1465 fluid, Q2/1401 fluid and perfume were mixed, and into this was homogenised a solution of propylene glycol, water, and the aluminium salt solution. Preferably the base has a viscosity of 400-10000cps at 25°C. The prepared base is then dosed in a conventional manner into an aerosol can with the required amount of propellant.

The cans used for the test were aluminium cans with an expoxyphenolic lacquered interior (ex Boxall), adapted to deliver a spray rate of 0.25-lgls, though other suitable can lacquers could include Mocoflex or PAM (ex Boxall).

The interior of the cans had been scratched with a spatula, and when the cans were dosed they were subjected to storage.

A series of cans were stored at 0°C, 20°C, and 40°C, the cans at 40°C being stored for 8 months, and those at 0° and 20°C being stored for 12 months. One can of each of the samples stored at each of the temperatures was opened weekly for the first four months and thereafter monthly, and inspected for signs of corrosion. None of the samples of examples 1-3 inspected showed any signs of pinholing or corrosion.

In contrast, identical compositions to those of examples 1-3 stored in tin plate cans having a lacquered interior showed signs of corrosion at 40°C after four months. In any event, these storage results were better than would have been expected if the composition without the volatile silicone and silicone surfactant had been stored in lacquered tin plate cans.

Also stored in scratched lacquered aluminium cans was a composition comprising 63% ethanol, 5% Reach 501, 1% fragrance, 1% isopropyl myristrate, and 30% CAP 30 propellant. Under similar storage conditions, these samples showed signs of corrosion after 5 weeks at 40°C.

## Claims

1. A propellant driven antiperspirant aerosol composition suitable for topical application to the human skin, comprising 10-50% by weight of a base and 50-90% by weight of a propellant, the base being in the form of a water in oil emulsion and comprising a dissolved aluminium salt, a volatile silicone, and a silicone surfactant, wherein the composition is packaged in an aluminium can.

2. An antiperspirant composition according to claim 1 comprising 10-40% by weight of a base and 60-90% by weight of a propellant.

3. An antiperspirant composition according to claim 1 or claim 2, wherein the volatile silicone is present in the composition at a level of 1-20% by weight of the composition.

4. An antiperspirant composition according to any of the preceding claims, wherein the silicone surfactant is a dimethicone copolyol silicone surfactant.

5. An antiperspirant composition according to any of the preceding claims, wherein the silicone surfactant is present in the composition at a level of 0.05-3% by weight.

6. An antiperspirant composition according to any of the preceding claims, wherein the composition comprises 2-25% by weight of water.

7. An antiperspirant composition according to any of the preceding claims, wherein the aluminium salt is present in the composition at a level of 0.5-25% by weight.

8. An antiperspirant composition according to any of the preceding claims, wherein the composition additionally comprises 0.5-10% by weight of an emollient.

9. An antiperspirant composition according to claim 8, wherein the emollient is a C₂-C₄ polyol emollient, especially propylene glycol or glycerol.

10. An antiperspirant composition according to any of the preceding claims, wherein the composition additionally comprises 1-10% by weight of a short chain C₁-C₆ monohydric alcohol.

## Patentansprüche

1. Treibmittel-getriebenes schweißhemmendes Aerosolmittel, geeignet zur örtlichen Anwendung auf der menschlichen Haut, umfassend 10-50 Gewichtsprozent einer Grundlage und 50-90 Gewichtsprozent eines Treibmittels, wobei die Grundlage in Form einer Wasser-in-Öl-Emulsion vorliegt und ein gelöstes Aluminiumsalz, ein flüchtiges Silikon und ein Silikontensid umfasst, wobei das Mittel in einer Aluminiumdose verpackt ist.

2. Schweißhemmendes Mittel nach Anspruch 1, umfassend 10-40 Gewichtsprozent einer Grundlage und 60-90 Gewichtsprozent eines Treibmittels.

3. Schweißhemmendes Mittel nach Anspruch 1 oder Anspruch 2, wobei das flüchtige Silikon in dem Mittel in einer Menge von 1-20 Gewichtsprozent des Mittels vorliegt.

4. Schweißhemmendes Mittel nach einem der vorangehenden Ansprüche, wobei das Silikontensid ein Dimethicon-Copolyol-Silikontensid ist.

5. Schweißhemmendes Mittel nach einem der vorangehenden Ansprüche, wobei das Silikontensid in dem Mittel in einer Menge von 0,05-3 Gewichtsprozent vorliegt.

6. Schweißhemmendes Mittel nach einem der vorangehenden Ansprüche, wobei das Mittel 2-25 Gewichtsprozent Wasser umfasst.

7. Schweißhemmendes Mittel nach einem der vorangehenden Ansprüche, wobei das Aluminiumsalz in dem Mittel in einer Menge von 0,5-25 Gewichtsprozent vorliegt.

8. Schweißhemmendes Mittel nach einem der vorangehenden Ansprüche, wobei das Mittel zusätzlich 0,5-10 Gewichtsprozent eines Erweichungsmittels umfasst.

9. Schweißhemmendes Mittel nach Anspruch 8, wobei das Erweichungsmittel ein C₂-C₄-Polyol-Erweichungsmittel, insbesondere Propylenglycol oder Glycerin, ist.

10. Schweißhemmendes Mittel nach einem der vorangehenden Ansprüche, wobei das Mittel zusätzlich 1-10 Gewichtsprozent eines kurzkettigen, einwertigen C₁-C₆-Alkohols umfasst.

## Revendications

1. Composition aérosol antitranspiration distribuée par un propulseur, appropriée pour une application topique sur la peau de l'être humain, comprenant de 10 à 50 % en masse d'une base et de 50 à 90 % en masse d'un propulseur, la base étant sous la forme d'une émulsion eau dans l'huile et comprenant un sel d'aluminium dissout, une silicone volatile et un agent tensioactif silicone, dans laquelle la composition est conditionnée dans un récipient en aluminium.

2. Composition antitranspiration selon la revendication 1, comprenant de 10 à 40 % en masse d'une base et de 60 à 90 % en masse d'un propulseur.

3. Composition antitranspiration selon la revendication 1 ou 2, dans laquelle la silicone volatile est présente dans la composition à un niveau allant de 1 à 20% en masse de la composition.

4. Composition antitranspiration selon l'une des revendications précédentes, dans laquelle l'agent tensioactif silicone est un agent tensioactif silicone de diméthicone copolyol.

5. Composition antitranspiration selon l'une des revendications précédentes, dans laquelle l'agent tensioactif silicone est présent dans la composition à un niveau allant de 0,05 à 3 % en masse.

6. Composition antitranspiration selon l'une des revendications précédentes, dans laquelle la composition comprend de 2 à 25 % en masse d'eau.

7. Composition antitranspiration selon l'une des revendications précédentes, dans laquelle le sel d'aluminium est présent dans la composition à un niveau allant de 0,5 à 25 % en masse.

8. Composition antitranspiration selon l'une des revendications précédentes, dans laquelle la composition comprend de façon additionnelle, de 0,5 à 10 % en masse d'un émollient.

9. Composition antitranspiration selon la revendication 8, dans laquelle l'émollient est un émollient polyol en C₂ - C₄, en particulier du propylène glycol ou du glycérol.

10. Composition antitranspiration selon l'une des revendications précédentes, dans laquelle la composition contient de façon additionnelle de 1 à 10 % en masse d'un alcool monohydrique à chaîne courte.
